# FASCICULE DE BREVET EUROPEEN

(11) **EP 2 931 924 B1**
(45) Date de publication et mention de la délivrance du brevet: **04.10.2017**
(21) Numéro de dépôt: 13818298.5
(22) Date de dépôt: 13.12.2013
(51) Int. Cl.: A61K 35/744, A61K 35/747, A61K 45/06, A61K 35/741, A61K 36/06, C12R 1/25, C12R 1/46, C12R 1/225

(54) **MÉTHODE POUR PRÉVENIR ET/OU TRAITER LES INFECTIONS, COLONISATIONS OU MALADIES LIÉES À STAPHYLOCOCCUS AUREUS, PSEUDOMONAS AERUGINOSA, STREPTOCOCCUS PYOGENES, ENTEROCOCCUS FAECIUM, ENTEROBACTER CLOACAE, PROTEUS MIRABILIS, BACTEROIDES FRAGILIS, STAPHYLOCOCCUS EPIDERMIDIS, PROPIONIBACTERIUM ACNES, CANDIDA ALBICANS ET/OU MALASSEZIA FURFUR**
VERFAHREN ZUR PRÄVENTION UND/ODER BEHANDLUNG VON INFEKTIONEN, KOLONISIERUNGEN ODER ERKRANKUNGEN IM ZUSAMMENHANG MIT STAPHYLOCOCCUS AUREUS, PSEUDOMONAS AERUGINOSA, STREPTOCOCCUS PYOGENES, ENTEROCOCCUS FAECIUM, ENTEROBACTER CLOACAE, PROTEUS MIRABILIS, BACTEROIDES FRAGILIS, STAPHYLOCOCCUS EPIDERMIDIS, PROPIONIBACTERIUM-ACNES, CANDIDA ALBICANS UND/ODER MALASSEZIA FURFUR
METHOD FOR PREVENTING AND/OR TREATING INFECTIONS, COLONISATIONS, OR ILLNESSES RELATED TO STAPHYLOCOCCUS AUREUS, PSEUDOMONAS AERUGINOSA, STREPTOCOCCUS PYOGENES, ENTEROCOCCUS FAECIUM, ENTEROBACTER CLOACAE, PROTEUS MIRABILIS, BACTEROIDES FRAGILIS, STAPHYLOCOCCUS EPIDERMIDIS, PROPIONIBACTERIUM ACNES, CANDIDA ALBICANS AND/OR MALASSEZIA FURFUR

(30) Priorité: 17.12.2012 FR 1262128
(43) Date de publication de la demande: 21.10.2015
(73) Titulaire: Urgo Recherche Innovation et Développement, 21300 Chenove (FR)
(72) Inventeur: DESROCHE, Nicolas, F-21800 Quetigny (FR); ARBAULT, Patrice, F-69530 Orlienas (FR); GUZZO, Jean, F-21121 Fontaine les Dijon (FR); RODRIGUES, Sandra, F-21800 Crimolois (FR); LAURENSOU, Christelle, F-21000 Dijon (FR); MERY, Sylvain, F-21560 Arc sur Tille (FR); APERT, Laurent, F-21000 Dijon (FR)
(74) Mandataire: Cabinet Plasseraud
(86) Numéro de dépôt international: PCT/FR2013/053071
(87) Numéro de publication internationale: WO 2014/096641

(56) Documents cités:
- EP-A1- 1 312 667
- WO-A1-2004/052462
- WO-A1-2008/074331
- WO-A1-2010/056198
- K. OKI ET AL: "Lactobacillus saniviri sp. nov. and Lactobacillus senioris sp. nov., isolated from human faeces", INTERNATIONAL JOURNAL OF SYSTEMATIC AND EVOLUTIONARY MICROBIOLOGY, vol. 62, no. Pt 3, mars 2012 (2012-03), pages 601-607, XP055074510, ISSN: 1466-5026, DOI: 10.1099/ijs.0.031658-0
- R. TEANPAISAN ET AL: "Inhibitory effect of oral Lactobacillus against oral pathogens", LETTERS IN APPLIED MICROBIOLOGY, vol. 53, no. 4, octobre 2011 (2011-10), pages 452-459, XP055047020, ISSN: 0266-8254, DOI: 10.1111/j.1472-765X.2011.03132.x

## Description

La présente invention se rapporte à des bactéries selon l'invention ayant des activités antagonistes (nommées « bactéries antagonistes selon l'invention » ci-après) de bactéries ou levures pathogènes appartenant aux genres et espèces *Staphylococcus aureus, Pseudomonas aeruginosa, Streptococcus pyogenes, Enterococcus faecium, Enterobacter cloacae, Proteus mirabilis, Bacteroides fragiles, Staphylococcus epidermidis, Propionibacterium acnes, Candida albicans* et/ou *Malassezia furfur* et à leur utilisation comme principe actif ou dans un dispositif médical, notamment dans le traitement et/ou la prévention de la colonisation et/ou des infections liées à ces bactéries ou levures pathogènes. Les bactéries selon l'invention, antagonistes des bactéries ou levures pathogènes ont été sélectionnées pour leurs facultés à inhiber l'adhésion et le développement et/ou la prolifération des bactéries ou levures pathogènes et ainsi stabiliser et/ou réguler l'écosystème. L'invention concerne des produits de soins contenant une ou plusieurs souches antagonistes selon l'invention, non pathogènes, destinée(s) à la prévention ou au traitement des infections ou colonisations au niveau de la peau, la plaie, des muqueuses et des phanères.

*Staphylococcus aureus* et *Pseudomonas aeruginosa* sont à l'origine d'un certain nombre de pathologies telles que les infections de la peau et des muqueuses, les infections des plaies, en particulier les plaies chroniques (ulcères, plaies diabétiques), les brûlures et les plaies chirurgicales. Ces espèces bactériennes sont notamment connues pour être à l'origine de retard de cicatrisation. Elles sont également impliquées dans d'autres pathologies telles que les infections gastro-intestinales, urinaires et pulmonaires, otites ou sinusites.

*Pseudomonas aeruginosa,* autrement connu sous le nom de bacille pyocyanique, peut, dans certaines conditions, être pathogène. Cette bactérie très résistante est - avec d'autres bactéries à gram-négatif - de plus en plus souvent impliquée dans des infections nosocomiales. Cette souche a la capacité de s'organiser en biofilm, ce qui la rend d'autant plus résistante aux antibiotiques. C'est l'une des bactéries les plus difficiles à traiter cliniquement. Le taux de mortalité atteint 50% chez les patients vulnérables (immunodéprimés). C'est un germe ubiquitaire, très résistant à de nombreux antiseptiques, fréquent en milieu hospitalier, et qui entraîne l'apparition (du fait de sa résistance aux antibiotiques) de véritables souches d'hôpital. Les formes de pathologie qu'elle engendre sont diverses : infection de l'oeil, infection des plaies (surtout brûlures et plaies opératoires), infection urinaire (surtout après sondages), infections gastro-intestinales et des poumons (par exemple après bronchoscopie), méningites d'inoculation, septicémies comme stade terminal d'infections graves, ou complication chez des malades soumis à un traitement immunosuppressif, des leucémiques, etc... Elle induit facilement des infections systémiques chez les immunodéprimés (par une chimiothérapie ou par le sida) et chez les victimes de brûlures et de fibrose kystique.

*Staphylococcus aureus* est l'espèce la plus pathogène du genre *Staphylococcus.* Elle est responsable d'intoxications alimentaires, d'infections localisées suppurées et, dans certains cas extrêmes, de septicémies physiques (greffe, prothèses cardiaques). L'espèce se révèle être pathogène opportuniste dans certains emplacements ou dans certaines circonstances. C'est un germe ubiquitaire qui possède une bonne résistance aux mécanismes d'épuration naturels. *S*. *aureus* se trouve également dans la flore commensale (chez 15 à 30 % des individus sains au niveau des fosses nasales et de la gorge, il peut également être retrouvé en faible quantité dans le tube digestif et souvent au niveau du périnée).

*S. aureus* possède des pouvoirs pathogènes, notamment un pouvoir invasif, une capacité à se multiplier et à se disséminer dans l'organisme, ainsi qu'un pouvoir toxique. *S*. *aureus* possède une grande capacité à développer des mutants résistants aux antibiotiques (à titre d'exemple, on peut citer le *Staphylococcus aureus* résistant à la méticilline, ou MRSA pour Methicillin-resistant *Staphylococcus aureus,* qui est une des causes principales d'infections nosocomiales). De ce fait, il partage avec le bacille pyocyanique le premier rôle dans les infections d'origine hospitalière.

Ces bactéries et levures pathogènes ont la capacité d'adhérer aux surfaces et notamment sur les tissus épithéliaux et de se développer sous la forme de biofilms chez les hôtes sains ou immunodéprimés. Les biofilms sont définis comme des ensembles complexes de micro-organismes (ou communautés microbiennes) adhérant à une surface biotique (tissus vivants, peau, etc.) ou abiotique (matériel inerte comme le silicone ou l'acier) et emprisonnés dans une matrice de polymères organiques (Costerton *et al.,* 1999, Dunne, 2002). Ces biofilms peuvent ainsi être mono-spécifiques c'est-à-dire composés d'une seule espèce bactérienne ou de levure, ou mixtes lorsqu'ils sont composés de plusieurs espèces bactériennes ou de levure.

Le traitement courant des infections liées à la présence d'espèces bactériennes pathogènes est l'utilisation d'antibiotiques (pénicilline, céphalosporine). Cependant l'utilisation de cette approche présente un certain nombre d'inconvénients :
- ces antibiotiques présentent souvent un spectre d'action important et peuvent être à l'origine de déséquilibre au niveau de la flore commensale, pouvant conduire par la suite à une colonisation par des germes pathogènes ;
- les bactéries sont capables d'acquérir des capacités de résistance à ces antibiotiques ;
- la résistance des bactéries sous la forme de biofilms est fortement augmentée par rapport à des bactéries seules, également appelées cellules planctoniques.

Face aux problèmes de résistances multiples des germes pathogènes aux antibiotiques, il existe un besoin urgent de trouver une alternative à ce type de traitement.

Une solution envisagée afin de prévenir ou traiter les infections d'origine bactérienne ou de levure est l'utilisation de souches bactériennes antagonistes des espèces pathogènes. On parle alors de bactériothérapie. Ces souches antagonistes sont capables, par leur métabolisme, de produire des molécules antimicrobiennes et/ou d'interférer avec l'adhésion des bactéries et levures pathogènes et/ ou de perturber la communication cellulaire entre les bactéries et levures pathogènes et/ ou de posséder des activités angiogéniques. Elles peuvent également contrôler l'inflammation (propriétés immuno-modulatrices). Ces bactéries forment un biofilm positif à la surface de la peau, des plaies, des muqueuses ou des phanères, qui peut s'installer temporairement et limiter l'implantation des bactéries et levures pathogènes.

Ceci est particulièrement intéressant lorsque l'infection et/ou la colonisation se situe au niveau de la peau et/ou d'une plaie.

Ces souches antagonistes appartiennent généralement à la famille des bactéries lactiques. Ces souches ont été isolées à partir de différentes matrices et principalement à partir de fèces. Les activités de ces souches sont largement utilisées pour la prévention et le traitement des désordres au niveau de la muqueuse intestinale ; on parle alors de bactéries probiotiques. D'autres applications au niveau de la muqueuse oto-rhino-laryngologée et de la muqueuse uro-génitale ont également été décrites. A titre d'exemple, on peut citer le brevet EP 871 503 qui porte sur une couche ou serviette hygiénique comprenant des microorganismes choisis parmi les genres *Lactobacillus curvatus, Lactobacillus plantarum* ou *Lactococcus lactis* possédant des propriétés antagonistes leur permettant de lutter contre des souches de microorganismes indésirables présentes ou se formant dans l'article absorbant, ou dans la zone urogénitale. La demande de brevet WO 99/53932 porte sur une composition pour le traitement des otites comprenant des microorganismes choisis parmi *Streptococcus sanguis, Streptococcus oralis* et *Streptococcus mitis.*

La demanderesse s'est particulièrement intéressée aux souches antagonistes des souches pathogènes *S. aureus* et *P. aeruginosa.*

Un des objets de la présente invention consiste potentiellement également à créer un biofilm positif transitoire à la surface de la peau, des phanères, des muqueuses et/ou des plaies, qui empêche ou limite l'implantation des pathogènes et leur prolifération.

Les bactéries antagonistes selon la présente invention sont particulièrement intéressantes pour leur application dans les pathologies impliquant les espèces pathogènes *S*. *aureus* et *P. aeruginosa* telles que les infections gastro-intestinales, urinaires et pulmonaires, otites ou sinusites, les plaies ou impliquant une colonisation des plaies, de la peau, des phanères ou des muqueuses.

Les bactéries antagonistes selon la présente invention sont particulièrement intéressantes pour leur application dans les différents types de plaies (chroniques, aigües, brûlures) mais également dans les affections cutanées (telles que, par exemple, les folliculites, impétigo, eczéma, furoncle, anthrax, panaris, atopies, perlèches, surinfections de lésions liées à des virus tels que celui de l'herpès ou de la varicelle).

En effet, une plaie est une lésion consécutive à un traumatisme, entraînant une perte cutanée ou une ouverture de la peau. En réponse à la formation d'une plaie, le processus de cicatrisation se met en place.

La cicatrisation naturelle d'une plaie se déroule principalement selon trois phases successives, chacune de ces phases étant caractérisée par des activités cellulaires spécifiques qui font progresser le processus de réparation selon des séquences chronologiques précises : la phase inflammatoire, la phase de granulation (ou phase proliférative), et la phase de formation d'une cicatrice. La rapidité et la qualité de la cicatrisation d'une plaie dépendent de l'état général de l'organisme atteint, de l'étiologie de la plaie, de l'état et de la localisation de la plaie, de la survenue ou non d'une infection, ainsi que des facteurs génétiques prédisposant ou non à des troubles de la cicatrisation. La peau arrachée ou coupée ne peut plus jouer son rôle de barrière contre les microbes qui peuvent alors pénétrer dans l'organisme provoquant une infection.

Les bactéries et les levures sont inévitablement présentes dans les plaies, il s'agit d'une colonisation naturelle.

Selon la quantité de bactéries et/ou de levures, les espèces bactériennes et/ou de levure présentes et la réponse de l'organisme, la distinction se fait entre colonisation, infection locale et infection.

La colonisation est la présence d'une certaine quantité de bactéries et/ou de levures au sein de la plaie sans que celle-ci n'entraîne une réponse inflammatoire. Après la multiplication des germes sur la plaie, leur adhérence aux cellules épithéliales, un équilibre s'instaure entre le patient et sa flore microbienne. Les germes restent à la surface de la plaie et y adhèrent pour former un biofilm. Sur le plan quantitatif, la colonisation est caractérisée par une quantité de germes inférieure à 10⁵ bactéries/mm³. Si la quantité de bactéries et/ou de levures dépasse ce chiffre, certains auteurs évoquent la colonisation critique, puis l'infection superficielle strictement locale en présence d'une colonisation bactérienne et/ou de levures importante. Cette présence importante de bactéries et/ou de levures nuit au bon déroulement du processus cicatriciel et induit un retard de cicatrisation.

On parlera d'infection quand la présence des germes bactériens et/ou de levures entraîne une réponse inflammatoire locorégionale et l'apparition de signes généraux avec des signes cliniques qui traduisent l'invasion tissulaire par les germes présents (grande quantité des germes présents, virulence bactérienne, diminution des mécanismes de défenses immunitaires du patient). L'infection est caractérisée par les signes cliniques locorégionaux et généraux.
Celle-ci peut conduire à l'extension de la plaie avec exposition de structures anatomiques sous-jacentes comme les ligaments ou les os.
La colonisation bactérienne ne demande pas de démarches thérapeutiques particulières tandis qu'une infection exige la mise en place de traitements antibactériens locaux et généraux. L'infection est généralement le facteur déterminant dans la non-cicatrisation ou le retard de cicatrisation de plaies, de façon directe ou indirecte. Toute contamination bactérienne et/ou par des levures d'une plaie majore l'inflammation.
Cela peut être bénéfique en cas de contamination modérée (inévitable en cas de plaie ouverte), mais devient délétère en cas d'infection de la plaie qui aboutit à un retard de cicatrisation.

Ainsi, la complication immédiate de la cicatrisation est avant tout l'infection qui empêche l'amorce du processus de cicatrisation de se mettre en place ; une plaie est considérée comme infectée lorsque la quantité de bactéries et/ou de levures présentes dans la plaie entrave le processus de cicatrisation ou aggrave la plaie.
Dans ce cas, la cicatrisation, dite de seconde intention, nécessite le plus souvent l'utilisation de médicaments dermatologiques et d'outils chirurgicaux (bistouri, curette, etc). Dans le cas d'un ulcère de jambe par exemple, il est nécessaire de désinfecter préalablement la plaie avec des antiseptiques (tels que la chlorhexidine ou l'hexamidine) puis d'effectuer un nettoyage, consistant à éliminer les débris et les excès de sécrétion, soit par geste chirurgical soit par des médicaments protéolytiques, i.e. dont le but est de détruire les lambeaux de peau morte qui viennent polluer la plaie. Parmi les actifs couramment utilisés pour traiter les infections des plaies, on peut également citer l'argent, le cuivre, l'octenidine, l'iode, le PHMB (polyhexaméthylène biguanide), le miel.

Cependant, les antibactériens et/ou antiseptiques ne sont pas recommandés dans les plaies. L'efficacité de ces actifs, eu égard à leur mécanisme d'action, est éphémère, ils sont inactivés par les substances organiques, ils peuvent être cytotoxiques vis-à-vis des éléments cellulaires, ils ont un spectre large, par conséquent, ils ne vont pas seulement s'attaquer aux bactéries pathogènes mais vont également détériorer la flore commensale. De plus, un certain nombre de souches bactériennes ont développé une résistance aux antiseptiques/antibactériens.

Il existe donc un besoin de disposer d'actifs efficaces dans le traitement et/ou la prévention d'infections ou de colonisations des plaies par les bactéries et/ou des levures qui soit efficace, facile d'utilisation et non invasif.

Une solution envisagée afin de prévenir ou traiter les infections ou colonisations d'origine bactérienne et/ou due à des levures au niveau de la peau, des plaies, des muqueuses et des phanères, est l'utilisation de souches bactériennes antagonistes des souches pathogènes. Ces souches sont capables, par leur métabolisme, de produire des molécules antimicrobiennes et/ou d'interférer avec l'adhésion des bactéries et/ou des levures pathogènes. Ces bactéries peuvent également former un biofilm positif à la surface de la peau, de la plaie, des muqueuses ou des phanères, qui peut s'installer temporairement et limiter l'implantation des bactéries et/ou des levures pathogènes.
Ces souches antagonistes appartiennent généralement à la famille des bactéries lactiques et/ou ont été isolées à partir de la flore commensale des muqueuses de l'Homme. Les activités de ces souches sont largement utilisées pour la prévention et le traitement des désordres au niveau de la muqueuse intestinale, de la muqueuse ORL et de la muqueuse vaginale. De telles souches sont décrites dans WO 96/38159, WO 00/61201, EP 1 140 226, EP 1 461 012 et WO 2006/013441.

L'effet antagoniste des souches est dû à différents mécanismes tels que :
▪ compétition nutritionnelle pour des substrats carbonés et/ou azotés ;
▪ production de molécules antimicrobiennes telles que l'acide lactique, le peroxyde d'hydrogène (H₂O₂), les molécules réductrices, les bactériocines ;
▪ compétition d'adhésion pour les sites de fixation sur les muqueuses (effet barrière) ;
▪ production de biosurfactants ;
▪ inhibition ou perturbation de la communication cellulaire entre les différentes espèces bactériennes ;
▪ activités immuno-modulatrices permettant la stimulation de l'immunité locale et systémique.

Les applications mettant en oeuvre des souches probiotiques au niveau de la peau et des plaies font généralement appel à l'une des activités décrites précédemment.

Nous pouvons ainsi citer des applications concernant des effets angiogéniques de souches probiotiques de *Lactobacillus acidophilus* ATCC 4356 et ATCC 43121, et *Bacillus polyfermenticus,* au niveau de la peau et de la muqueuse intestinale (Halper *et al.,* 2003, Im *et al.,* 2009). Des souches probiotiques de *Bifidobacterium longum router,* de *Lactobacillus paracasei* CNCM I-2116, de *Lactobacillus johnsonii* La1 et de *Vitreoscilla filiformis,* sous formes topiques, ont également été utilisées pour réguler des phénomènes d'inflammation et des dérèglements au niveau de la peau (Gueniche *et al.,* 2006, Gueniche *et al.,* 2008a, Gueniche *et al.,* 2008b, Gueniche *et al.,* 2008c, Gueniche *et al.,* 2008d, Gueniche *et al.,* 2009, Gueniche *et al.,* 2010). Dans le cadre de ces applications, les auteurs mettent en avant le pouvoir immuno-modulateur des souches. De telles souches sont décrites dans les demandes de brevet EP 1 593 382 et WO 2006/037922.

L'utilisation d'une souche de *Lactobacillus plantarum* ATCC 10241 a été décrite pour limiter la croissance de *Pseudomonas aeruginosa* et la formation de biofilms par cette souche en libérant des molécules signaux perturbant la communication cellulaire (ou « quorum-sensing »). L'application directe d'un morceau de gaze imbibé avec une culture de cette souche, sur des modèles de brûlures chez le rat et sur des brûlures humaines, a permis de démontrer son efficacité (Peral *et al.,* 2009, Valdez *et al.,* 2005). Le pouvoir immuno-modulateur de cette souche vis-à-vis des neutrophiles et des leucocytes a également été exploité pour traiter des plaies chroniques (plaies diabétiques, ulcères veineux) et pour réduire l'inflammation causée par *P. aeruginosa* (Peral *et al.,* 2010, Ramos *et al.,* 2010, Ramos *et al.,* 2012). Une formulation de cette souche dans un film d'alginate a été décrite par Brachkova *et al.* (Brachkova *et al.,* 2011) pour la prévention des infections de brûlures par *P. aeruginosa.*

Une souche de *Lactobacillus fermentum* RC-14 a démontré des capacités remarquables pour inhiber l'adhésion de *Staphylococcus aureus* et limiter les infections liées à ce germe au niveau d'implants chirurgicaux (Gan *et al.,* 2002). Cet effet antimicrobien a été attribué à la libération d'un biosurfactant.

L'efficacité de plusieurs souches de *Lactobacillus* et *Bifidobacterium* isolées à partir de sources différentes, dans la prévention et le traitement de *Staphylococcus aureus* résistant à la méticilline, a également été démontrée dans une étude de H. Sikorska et al., 2013.

L'inhibition de la croissance de *Staphylococcus epidermidis* et *Propionibacterium acnes* par des souches de *Lactobacillus reuteri* (KCTC 3594, KCTC 3678 et KCTC 3679) a été mise en évidence (Kang *et al.,* 2012).

L'effet des bactériocines produites par *Lactococcus sp.* HY 449 contre les bactéries inflammatoires de la peau telles que *Staphylococcus epidermidis* ATCC 12228, *Staphylococcus aureus* ATCC 65389, *Streptococcus pyogenes* ATCC 21059, et *Propionibacterium acnes* ATCC 6919 a été démontré (Ho *et al.,* 2006)

Des pansements ou des tissus faisant appel à la mise en oeuvre de souches probiotiques (bactéries lactiques ou *Bacillus coagulans*) ont déjà été décrits (US 7,025,974 et WO 2008/074331). L'efficacité de ces formulations repose uniquement sur l'utilisation de souches bactériennes capables de produire de l'acide lactique comme principal agent antimicrobien à large spectre.

La présente invention a pour but de proposer de nouveaux actifs à base de bactéries antagonistes selon l'invention des bactéries pathogènes *Staphylococcus aureus* et/ou *Pseudomonas aeruginosa,* qui soient efficaces dans le traitement et/ou la prévention de la colonisation et/ou des infections liées à ces bactéries pathogènes, notamment au niveau de la peau, de la plaie, des muqueuses et des phanères. En outre, ces actifs peuvent être administrés directement au contact de la peau, de la plaie, des muqueuses et des phanères, ou peuvent facilement être incorporés dans des compositions, notamment des compositions convenant pour une application topique, et sont non invasifs.

De manière innovante, les bactéries antagonistes selon l'invention ont été préalablement sélectionnées sur la base de plusieurs critères : (i) leurs capacités à inhiber la croissance des germes pathogènes que sont *S. aureus* et *P. aeruginosa,* (ii) leurs capacités à adhérer et à former un biofilm positif sur du collagène et de l'épiderme, (iii) leurs capacités à limiter l'adhésion des germes pathogènes sur des matrices contenant du collagène et (iv) leurs capacités à limiter la réaction inflammatoire (production de TNF-α par les macrophages).

Ces bactéries selon l'invention sont capables de développer un effet barrière au niveau de la peau, de la plaie, des muqueuses et des phanères et ainsi de prévenir et limiter les risques de colonisations et d'infections.

Les inventeurs se sont particulièrement intéressés à sélectionner des souches antagonistes principalement de *S. aureus* et *P. aeruginosa,* qui sont les espèces pathogènes majoritairement impliquées dans les infections, notamment les infections nosocomiales.

De manière complémentaire, les inventeurs ont sélectionné des souches bactériennes selon l'invention qui, en plus d'inhiber des souches de *S. aureus* et *P. aeruginosa,* inhibent le développement d'autres espèces pathogènes telles que *Streptococcus pyogenes, Enterococcus faecium, Enterobacter cloacae, Proteus mirabilis, Bacteroides fragilis, Staphylococcus epidermidis, Propionibacterium acnes, Candida albicans* et *Malassezia furfur.* Ces germes sont plus particulièrement impliqués dans les infections des plaies et de la peau.

*Propionibacterium acnes* est un bacille à Gram positif anaérobie, de croissance relativement lente et normalement présent dans la peau, les cheveux et les muqueuses. Il est responsable de l'acné.

La bactérie est présente largement dans la flore cutanée de la plupart des adultes en bonne santé mais elle est aussi responsable d'infections post-opératoires, notamment en cas de présence d'implant, potentiellement sévères : infections du système nerveux central, endophtalmies, endocardites, infections de la sphère ORL et pulmonaire, spondylodiscites, péritonites et infections ostéo-articulaires.

*Le Staphylococcus epidermidis* est un commensal de la peau chez pratiquement 100 % des humains ; ses propriétés lipolytiques lui permettent de prospérer dans le sébum. Il est normalement inoffensif, mais il provoque d'authentiques infections chez les patients dont le système immunitaire est compromis ou des patients qui ont des cathéters, des prothèses. Ce peut être des infections dermatologiques et des infections nasales comme des sinusites ou encore des infections urinaires chez la femme et plus rarement chez l'homme. Ces bactéries ont la capacité de produire des biofilms qui leur permettent d'adhérer aux surfaces des prothèses médicales.
*Candida albicans* se manifeste différemment selon sa localisation. Chez les patients immunocompétents (i.e. dont le système de défense est efficace, au contraire des immunodéprimés), il apparait sous forme de muguet au niveau buccal, rougeurs et démangeaisons au niveau cutané, essentiellement dans les plis, zones chaudes et humides favorables au développement de la levure, de petites inflammations locales génitales comme urétrite chez l'Homme ou vulvo-vaginite avec pertes blanchâtres et démangeaisons chez la femme.
*Malassezia furfur* est une levure impliquée principalement dans la dermite séborrhéique qui est une dermatose inflammatoire cutanée fréquente (observée dans 3 % à 5 % de la population). Elle se présente sous la forme de plaques rouges, recouvertes de squames grasses et jaunâtres, plus ou moins prurigineuses, prédominant dans les zones riches en glandes sébacées, les zones séborrhéiques. A la face, la topographie des lésions est évocatrice : sillon entre le nez et les lèvres, racine des sourcils, cuir chevelu, ailes du nez, plis des pavillons, conque des oreilles, conduits auditifs externes. Au cuir chevelu, l'atteinte fréquente se traduit par un état pelliculaire plus ou moins séborrhéique. Sur le tronc, on remarque deux zones fréquentes chez l'Homme : le sternum et la région entre les deux omoplates.
On la rencontre à la fois chez l'adulte et le nourrisson (de moins de 3 mois), chez qui elle se signale par les classiques « croûtes de lait » dans le cuir chevelu et un érythème fessier. Chez l'adulte, cette pathologie s'observe surtout sur des sujets âgés entre 20 et 40 ans. Les hommes sont plus fréquemment atteints que les femmes. Chez les femmes, le développement s'observe plus particulièrement au moment de la ménopause. La pathologie d'origine inflammatoire, multifactorielle n'est pas contagieuse et peut évoluer par poussées déclenchées le plus souvent par le stress ou la pollution et l'absence de soleil.
La cause est inconnue, mais un champignon microscopique jouerait un rôle sur un terrain immuno-allergique particulier.
Le Pityriasis versicolore est une mycose superficielle, liée à la prolifération sur la peau de *Malassezia furfur.* Cette levure vit normalement à la surface de la peau mais dans certaines situations, elle se multiplie à grande vitesse et occasionne ces petites taches brunes ou décolorées.
Ce champignon affectionne particulièrement les peaux grasses, siégeant sur le thorax mais également sur le cou et les épaules, le haut du dos et sur les membres supérieurs, rarement sur les membres inférieurs.

La présente invention a donc pour objet une bactérie choisie parmi les souches de *Lactobacillus saniviri* également désignée dans la présente demande par F3C5p (enregistrée le 12 juillet 2012 sous le n° CNCM I-4650 à la Collection Nationale de Culture de Microorganismes, Institut Pasteur, 25 rue du Docteur Roux, 75724 Paris Cedex 15, France, ci-après désignée « CNCM »), *Lactobacillus salivarius* également désignées dans la présente demande par F50C2p, F52C3p et F41C3p (enregistrées respectivement sous les n° CNCM I-4651, CNCM I-4652 et CNCM I-4653 à la CNCM), *Streptococcus mitis* également désignée dans la présente demande par F3C2v (enregistrée le 12 juillet 2012 sous le n° CNCM I-4654 à la CNCM) et *Lactobacillus pentosus* ou *plantarum* également désignée dans la présente demande par L1C1 (enregistrée le 12 juillet 2012 sous le n° CNCM I-4655 à la CNCM). Ces bactéries sont appelées « bactéries selon l'invention » dans la présente demande.

La présente invention a également pour objet une bactérie selon l'invention pour son utilisation comme immuno-modulateur.

La présente invention a également pour objet l'utilisation d'une telle bactérie selon l'invention comme principe actif ou comme dispositif médical ou comme cosmétique ou encore comme complément alimentaire. La bactérie selon l'invention peut également être utilisée comme principe actif au sein d'un médicament, d'un dispositif médical, d'un cosmétique ou d'un complément alimentaire.

La présente invention vise également une bactérie selon l'invention pour son utilisation dans le traitement et/ou la prévention de la colonisation et/ou des infections liées à au moins une bactérie ou une levure choisie parmi *P. aeruginosa, S. aureus, S. pyogenes, E. faecium, E. cloacae, P. mirabilis,B. fragiles, Staphylococcus epidermidis, Propionibacterium acnes, Candida albicans* et *Malassezia furfur.*

Préférentiellement, la présente invention vise une bactérie selon l'invention pour son utilisation dans le traitement et/ou la prévention de la colonisation et/ou des infections liées à *P. aeruginosa* et/ou *S. aureus.*

La présente invention porte plus particulièrement sur le traitement et/ou la prévention par les bactéries selon l'invention de la colonisation et/ou des infections des plaies et de la peau par les bactéries ou les levures pathogènes.

Le terme « traitement » ou « traiter » une infection ou une colonisation signifie diminuer et/ou inhiber le développement de cette infection ou colonisation.

Le terme « prévention » ou « prévenir » une infection ou colonisation signifie diminuer et/ou éviter l'apparition des symptômes de l'infection ou de la colonisation.

Par « infection ou colonisation des plaies », on entend une infection ou colonisation choisie parmi les ulcères du pied diabétique, les ulcères de jambe d'origine artérielle, les ulcères de jambe d'origine veineuse, les escarres, les panaris, les infections liées aux plaies aiguës, les infections liées aux plaies traumatiques, telles que les plaies par section, les plaies pénétrantes, les plaies par agents thermiques ou caustiques et les brûlures ; et les infections liées aux plaies post-opératoires, telles que les plaies suturées simples d'une incision chirurgicale, les plaies suturées complexes après exérèse cutanée, les dermabrasions chirurgicales et les plaies non suturables nécessitant une cicatrisation par seconde intention.

La bactérie selon l'invention peut être incorporée dans une composition, comme par exemple un dispositif médical. Par dispositif médical, on entend au sens de la présente invention les prothèses, cathéters, pansements, poches d'ostomie, champs chirurgicaux, sondes urinaires, sondes endotrachéales, drains trans-tympaniques, bandes, bandes de contention, implants orthopédiques et mammaires, lentilles de contact, dispositifs intra-utérins, ou encore les matériels pour sutures.

La présente invention a également pour objet une composition comprenant au moins une bactérie selon l'invention. Ladite composition comprend, dans un milieu acceptable, au moins une bactérie selon l'invention. Par milieu acceptable, on entend un milieu compatible avec la peau, la plaie, les muqueuses et les phanères.

La composition selon l'invention, ou la bactérie selon l'invention, peut être administrée par voie topique, orale ou parentérale.
De préférence, une telle composition ou une telle bactérie selon l'invention est adaptée pour une application topique sur la peau, la plaie, les phanères ou les muqueuses comme la muqueuse rhino-paryngée, uro-génitale, digestive, ou respiratoire. La composition peut être ainsi directement appliquée sur la peau, la plaie ou les muqueuses.
Par application topique, on entend une application sur la peau, la plaie, les muqueuses et/ou les phanères.

La composition selon l'invention comprend de préférence 10³ à 10¹² bactéries selon l'invention, de préférence 10⁶ à 10¹¹.

La composition topique peut se présenter sous forme liquide, pâteuse ou solide, et plus particulièrement sous forme d'onguent, de crème, de lait, de pommade, de poudre, de tampon imbibé, de syndet, de lingette, de solution, de gel, de spray, de mousse, de suspension, de lotion, de stick, de shampoing ou de base lavante. Elle peut également se présenter sous forme de suspension de microsphères ou nanosphères ou de vésicules lipidiques ou polymériques ou de microcapsules ou de patch polymérique et d'hydrogel permettant une libération contrôlée. Cette composition pour application topique peut se présenter sous forme anhydre, sous forme aqueuse ou sous forme d'émulsion.

La composition parentérale peut se présenter sous forme d'une solution pour injection sous-cutanée (solution injectable).

La composition orale peut se présenter sous forme d'une solution, d'une poudre, d'une gélule, d'un comprimé, ou peut être intégrée à un produit alimentaire tel qu'un produit laitier.

La bactérie selon l'invention peut être incorporée à la composition sous forme de cellule inactivée, notamment inactivée par la chaleur, par les UV ou par tout autre processus. Elle peut également être incorporée sous forme de cellule vivante, encapsulée ou non, immobilisée ou non, lyophilisée ou non, ou encore sous forme d'extrait cellulaire. Il peut également s'agir de lysat cellulaire ou de tout autre type de présentation connu de l'homme du métier.

De manière préférentielle, les bactéries antagonistes utilisées dans le cadre de la présente invention, ou une formulation galénique les contenant, seront appliquées directement au niveau de la plaie soit sous forme de composition topique soit intégrées à une matrice synthétique ou non entrant dans la composition d'un pansement.
Dans ce mode de réalisation, les bactéries selon l'invention sont formulées dans un pansement, ou bien formulées dans une composition qui est elle-même incluse dans un pansement ou peut être incluse au moment de l'application du pansement. Le pansement selon l'invention comprend donc au moins une bactérie selon l'invention, ou au moins une composition selon l'invention.

Les bactéries antagonistes selon l'invention ou les compositions selon l'invention les contenant, pourront être incorporées dans un élément quelconque de la structure d'un pansement, sous réserve que lesdites bactéries selon l'invention puissent entrer directement ou indirectement en contact avec la surface de la plaie.

De préférence et afin de favoriser une action rapide, la bactérie selon l'invention (ou une composition selon l'invention la contenant) sera incorporée dans la couche du pansement qui vient en contact avec la plaie, ou sera déposée sur la surface du pansement qui vient en contact avec la plaie.
Avantageusement, la bactérie selon l'invention (ou une composition selon l'invention la contenant) pourra ainsi être déposée, de façon continue ou discontinue, sur la surface destinée à venir au contact de la plaie :
- soit sous forme liquide, par exemple par vaporisation d'une solution ou suspension la contenant ;
- soit sous forme solide, par exemple par tamisage d'une poudre la contenant.

Par pansement, on entend désigner, au sens de la présente demande, tous types de pansements utilisés pour le traitement des plaies. Typiquement, un pansement comprend au moins une couche ou matrice, adhésive ou non.

La couche ou surface venant en contact avec la plaie pourra être constituée de tout matériau ou association de matériaux habituellement utilisé(s) à cet effet dans le domaine des pansements. Parmi ces matériaux, on peut citer les mousses absorbantes, en particulier les mousses hydrophiles à base de polyuréthane ; des matériaux textiles, en particulier des non tissés à base de fibres absorbantes ou superabsorbantes ; des hydrogels ; ou une combinaison de ces matériaux; un matériau adhésif absorbant ou non ; une structure interface adhérente ou non.

De façon générale, on pourra jouer sur la galénique ou la structure du pansement pour obtenir un profil de relargage spécifique de la bactérie selon l'invention, rapide ou retardé, selon les besoins.

Ainsi, la bactérie selon l'invention (ou une composition selon l'invention la contenant) pourra être intégrée à tout type de pansement existant tel que, sans que ces exemples soient limitatifs :
- les alginates tels que, à titre d'exemples, les produits commercialisés sous les dénominations Urgosorb® par les Laboratoires URGO ou Melgisorb® par Mölnlycke ;
- les hydrocellulaires tels que, à titre d'exemple, les produits commercialisés sous les dénominations Urgotul Absorb® par les Laboratoires URGO ou Tielle par Systagenix ;
- les pansements hydrocolloïdes tels que, à titre d'exemples, les produits commercialisés sous les dénominations Algoplaque® par les Laboratoires URGO et Comfeel® par la société Coloplast ;
- les hydrogels tels que, à titre d'exemple, les produits commercialisés sous les dénominations Urgo Hydrogel® par les Laboratoires URGO et Intrasite Gel® par Smith & Nephew.
- Les pansements liquides tels que, à titre d'exemple, les produits commercialisés sous les dénominations Urgo Crevasses par les Laboratoires URGO ou Nexcare® par 3M.

Bien entendu, la quantité de bactérie(s) selon l'invention utilisée dans la formulation galénique ou dans le pansement sera adaptée en fonction de la cinétique recherchée ainsi que des contraintes spécifiques liées à sa nature, solubilité, résistance à la chaleur, etc.

Dans le cadre de son utilisation dans un élément de pansement, la(es) bactérie(s) selon l'invention sera(ont) incorporée(s) en une quantité telle que la quantité de bactéries selon l'invention relarguées dans les exsudats de la plaie soit comprise entre 0,001 g/l et 50 g/l, et de préférence entre 0,01 et 10 g/l.

Selon la galénique choisie, la mise en oeuvre du pansement pourra nécessiter une imbibition ou hydratation préalable du pansement avec une solution, comme par exemple du sérum physiologique, afin d'activer les bactéries antagonistes selon l'invention.

La composition selon l'invention peut comprendre une ou plusieurs souches antagonistes selon l'invention, associée(s) ou non à au moins un composé choisi parmi les probiotiques, les prébiotiques et les levures. Parmi les prébiotiques, on peut citer, à titre d'exemple, les fructanes tels que l'inuline, les fructo-oligosides ou les trans-galactooligosaccharides, ou encore les sucres à chaîne longue ou chaîne ramifiée.

La bactérie selon l'invention peut également être associée à des agents actifs notamment choisis parmi les agents antifongiques, les anti-douleurs, les anti-inflammatoires, les agents favorisant la cicatrisation, les agents hydratants, les agents kératolytiques, les actifs restructurants, et les anesthésiques.
En particulier, les actifs pouvant être introduits dans la composition selon l'invention peuvent être choisis parmi :
- les antifongiques tels que les polyènes, le nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole,Posaconazole, Voriconazole), les allylamines, la Terbinafine, l'Amorolfine, la Naftifine, ou la Buténafine ;
- la flucytosine (antimétabolite), la Griséofulvine, la Caspofungine, ou la Micafungine ;
- les anti-douleur tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, ou l'acide méfénamique ;
- les actifs favorisant la cicatrisation tels que le Rétinol, la Vitamine A, la Vitamine E, la N-acétyl-hydroxyproline, les extraits de Centella Asiatica, la papaïne, les silicones, les huiles essentielles de thym, de niaouli, de romarin et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, ou l'Allantoïne ;
- les agents hydratants tels que l'acide hyaluronique, l'urée, le glycérol, les acides gras, modulateurs des aquaporines, les huiles végétales, le chitosan, certains sucres dont le sorbitol, les beurres et les cires ;
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline® (Sederma), les protéases obtenues par fermentation de Bacillus Subtilis, ou le produit Linked-Papain® (SACI-CFPA);
- les actifs restructurants (par exemple restructurants des phanères) tels que les dérivés de silice, la vitamine E, la camomille, le calcium, l'extrait de prêle, ou le Lipester de soie;
- les anesthésiques tels que la benzocaïne, la lidocaïne, la dibucaïne, le chlorhydrate de pramoxine, la bupivacaïne, la mepivacaïne, la prilocaïne, ou l'étidocaïne.

Il va être donné, à titre d'illustration et sans aucun caractère limitatif, divers exemples d'utilisation des bactéries selon l'invention, qui permettent de démontrer les activités antibactériennes des souches antagonistes selon l'invention.
La figure 1 représente les halos d'inhibition observés après contact des bactéries selon l'invention avec des bactéries pathogènes sur milieu gélosé après 48 heures d'incubation.
La figure 2 représente un halo d'inhibition formé après contact des bactéries selon l'invention, incorporées dans un pansement, avec des bactéries pathogènes sur milieu gélosé après 48 heures d'incubation.
La figure 3 représente la capacité des bactéries selon l'invention à adhérer sur des épidermes reconstitués (en quantité de bactéries adhérées UFC/cm²).
La figure 4 représente les résultats des tests de compétition d'adhésion sur des surfaces collagène des bactéries selon l'invention vis-à-vis de *S. aureus* (quantité de *S. aureus* adhérée après 24h d'incubation (UFC/cm²)).
La figure 5 représente les résultats des tests d'exclusion sur des surfaces collagène des bactéries selon l'invention vis-à-vis de *S. aureus* (quantité de *S. aureus* après 24h d'incubation (UFC/cm²)).
La figure 6 représente les résultats des tests de compétition d'adhésion sur des surfaces collagène des bactéries selon l'invention vis-à-vis de *P. aeruginosa* (quantité de *P.*
*aeruginosa* adhérée après 24h d'incubation (UFC/cm²)).
La figure 7 représente les résultats des tests d'exclusion sur des surfaces collagène des bactéries selon l'invention vis-à-vis de *P. aeruginosa* (quantité de *P. aeruginosa* après 24h d'incubation (UFC/cm²)).

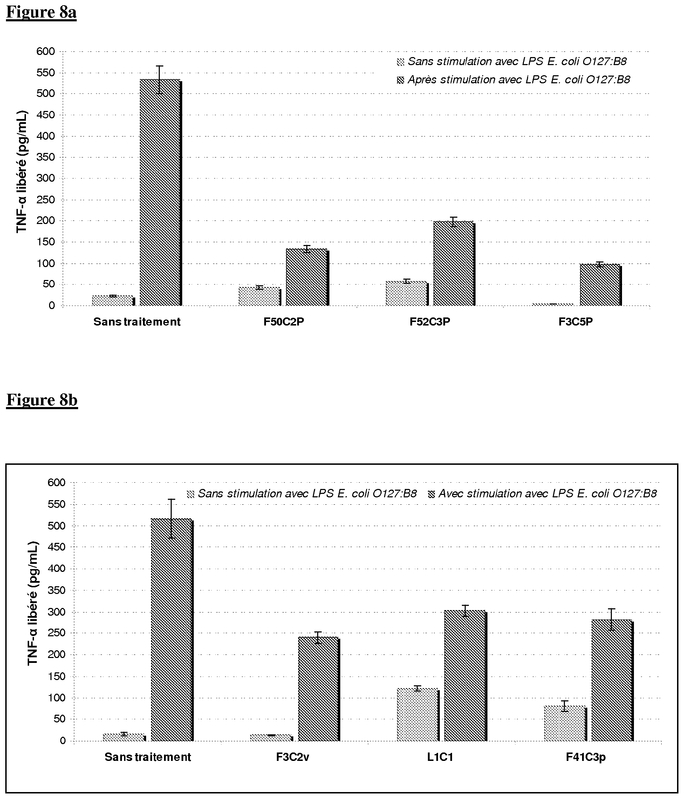

Les figures 8a et 8b représentent la quantité de TNF-á libéré (pg/mL) par les macrophages après 3,5 heures de contact avec les bactéries selon l'invention, sans ou avec stimulation par du LPS d'*E. coli* 0127 :B8.

### EXEMPLE 1 : Activités antimicrobiennes des souches de bactéries Lactobacillus saniviri F3C5p (CNCM I-4650), Lactobacillus salivarius F50C2p (CNCM I-4651), Lactobacillus salivarius F52C3p (CNCM I-4652), Lactobacillus salivarius F41C3p (CNCM I-4653), Streptococcus mitis F3C2v (CNCM I-4654) et Lactobacillus pentosus ou plantarum L1C1 (CNCM I-4655)

Les bactéries *Lactobacillus saniviri* F3C5p (CNCM I-4650), *Lactobacillus salivarius* F50C2p, F52C3p et F41C3p (respectivement CNCM I-4651, CNCM I-4652 et CNCM I-4653), *Streptococcus mitis* F3C2v (CNCM I-4654) et *Lactobacillus pentosus* ou *plantarum* L1C1 (CNCM I-4655) ont été cultivées dans du milieu de Man Rogosa Sharpe (MRS) pendant 16 heures, à 37°C et en anaérobiose. Des gouttes de 10 µL de ces cultures ont été déposées à la surface de milieux gélosés Tryptic Soy Agar (TSA) préalablement ensemencés dans la masse avec 10⁶ à 10⁷ cellules de *Staphylococcus aureus* résistante à la méthicilline (MRSA) ATCC 43300 ou *Pseudomonas aeruginosa* ATCC 9027 ou *Bacteroides fragilis* ATCC 23745 ou *Enterobacter cloacae* CIP 105132 ou *Enterococcus faecium* ATCC 700221 ou *Proteus mirabilis* CIP 107283 ou *Streptococcus pyogenes* ATCC 19615 ou *Staphylococcus epidermidis* ATCC 14990 ou *Propionibacterium acnes* ATCC 6919 ou *Candida albicans* ATCC 10231 ou *Malassezia furfur* ATCC 14521 par mL de milieu gélosé. Un témoin négatif a été réalisé en déposant 10 µL de milieu MRS stérile.
Les boîtes ont été incubées en anaérobiose à 37°C pendant 24 et 48 heures. Après incubation, le diamètre des halos d'inhibition formés autour des gouttes (en mm) a été mesuré.

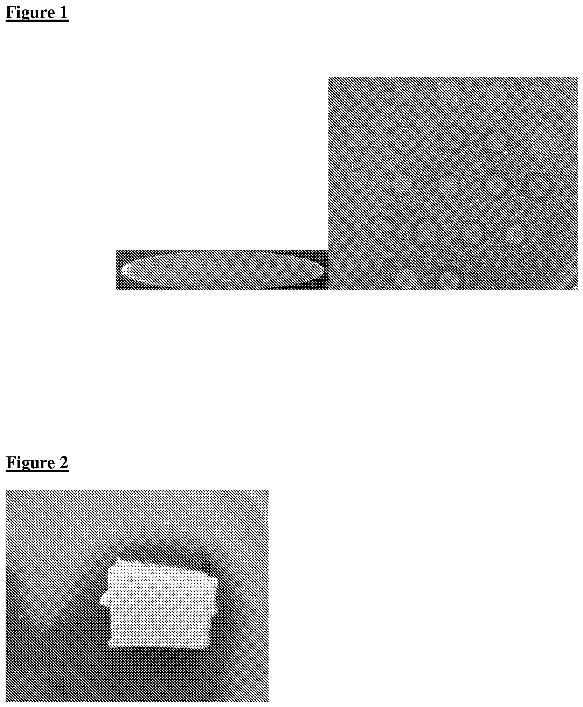

Les résultats de cette expérience figurent dans le tableau 1.
Le signe « - » indique une absence d'halo d'inhibition, un signe « + » indique un halo d'inhibition de diamètre inférieur à 1 mm, un signe « ++ » indique un halo d'inhibition de diamètre compris entre 1 mm et 3 mm et un signe « +++ » indique un halo d'inhibition de diamètre supérieur à 3 mm. Aucun halo d'inhibition n'a été mis en évidence avec le témoin négatif. Des halos d'inhibition ont été observés avec les 3 souches de bactéries testées (cf. Figure 1).

**Tableau 1 : Résultats des tests d'activité antimicrobienne des souches Lactobacillus saniviri F3C5p (CNCM I-4650), Lactobacillus salivarius F50C2p (CNCM I-4651), Lactobacillus salivarius F52C3p (CNCM I-4652), Lactobacillus salivarius F41C3p (CNCM I-4653), Streptococcus mitis F3C2v (CNCM I-4654) et Lactobacillus pentosus ou plantarum L1C1 (CNCM I-4655)**

| **Souches** | ***S*. *aureus*** ATCC 43300 | ***P. aeruginosa*** ATCC 9027 | ***B. fragilis*** ATCC 23745 | ***E. cloacae*** CIP105132 | ***P. mirabilis*** CIP 107283 | ***E. faecium*** ATCC 700221 | ***S. pyogenes*** ATCC 19615 | ***Staphylococcus epidermidis*** ATCC 14990 | ***Propionibacterium acnes*** ATCC 6919 | ***Candida albicans*** ATCC 10231 | ***Malassezia furfur*** ATCC 14521 |
|---|---|---|---|---|---|---|---|---|---|---|---|
| ***L. saniviri* F3C5p** (CNCM I-4650) | +++ | + | +++ | ++ | ++ | +++ | +++ | +++ | +++ | + | +++ |
| ***L. salivarius* F50C2p** (CNCM 1-4651) | ++ | + | ++ | + | + | ++ | +++ | +++ | +++ | - | - |
| ***L**.* ***salivarius* F52C3p** (CNCM 1-4652) | ++ | ++ | ++ | + | ++ | ++ | +++ | +++ | +++ | - | - |
| ***L**.* ***salivarius* F41C3p** (CNCM 1-4653) | + | - | - | + | + | - | +++ | ++ | +++ | - | - |
| ***S***. ***mitis* F3C2v** (CNCM 1-4654) | ++ | - | +++ | - | ++ | +++ | ++ | - | +++ | - | ++ |
| ***Lactobacillus sp.* L1C1** (CNCM 1-4655) | + | - | ++ | + | + | + | ++ | + | +++ | - | - |

### EXEMPLE 2 : Activités antimicrobiennes des souches de bactéries Lactobacillus saniviri F3C5p (CNCM I-4650), Lactobacillus salivarius F50C2p (CNCM I-4651), Lactobacillus salivarius F52C3p (CNCM I-4652), Lactobacillus salivarius F41C3p (CNCM I-4653), Streptococcus mitis F3C2v (CNCM I-4654) et Lactobacillus pentosus ou plantarum L1C1 (CNCM I-4655) après incorporation dans un pansement

Les bactéries F3C5p, F50C2p, F52C3p, F41C3p, F3C2v et L1C1 ont été cultivées dans du milieu de Man Rogosa Sharpe (MRS) pendant 16 heures, à 37°C et en anaérobiose. Des morceaux de pansements (1 cm × 1 cm) composés de mousse de polyuréthane et d'une trame lipido-colloïde ont été imbibés avec 500 µL de la culture de bactéries selon l'invention (concentration comprise entre 10⁹ et 10¹⁰ UFC.mL⁻¹) et déposés à la surface de milieux gélosés Tryptic Soy Agar (TSA) préalablement ensemencés dans la masse avec 10⁶ à 10⁷ cellules de *Staphylococcus aureus* MRSA ATCC 43300 ou *Pseudomonas aeruginosa* ATCC 9027. Un témoin négatif a été réalisé en déposant 500 µL de milieu MRS stérile.

Les boîtes ont été incubées en anaérobiose à 37°C pendant 24 et 48 heures. Après incubation, l'activité antimicrobienne est mise en évidence par la présence d'un halo d'inhibition. Après incubation, des halos d'inhibition sont visibles autour des pansements imbibés avec les bactéries *Lactobacillus saniviri* F3C5p, *Lactobacillus salivarius* F50C2p, *Lactobacillus salivarius* F52C3p, *Lactobacillus salivarius* F41C3p, *Streptococcus mitis* F3C2v et *Lactobacillus pentosus* ou *plantarum* L1C1 (Cf. figure 2). Aucun halo d'inhibition n'est visible autour des pansements imbibés avec le milieu MRS.

### EXEMPLE 3 : Capacités des souches selon l'invention à adhérer sur du collagène

Des essais d'adhésion des bactéries *Lactobacillus saniviri* F3C5p, *Lactobacillus salivarius* F50C2p, *Lactobacillus salivarius* F52C3p, *Lactobacillus salivarius* F41C3p, *Streptococcus mitis* F3C2v et *Lactobacillus pentosus* ou *plantarum* L1C1 ont été menés sur des épidermes reconstitués de type EpiSkin (SkinEthic) (1,07 cm2) âgés de 13 jours. Les inserts contenant les épidermes ont été placés dans une plaque de 12 puits avec 2 mL de milieu de maintenance et incubés pendant 24 heures à 35°C ± 2°C afin de régénérer les épidermes.

Après incubation, le milieu de maintenance a été retiré et 2 mL de milieu MRS ont été ajoutés. Un milieu simulant les exsudats de plaie, nommé Simulated Wound Fluid (50/50 vol/vol) (décrit dans Werthén et al, 2010) a ensuite été inoculé avec les bactéries selon l'invention (concentration d'environ 2,5 × 10⁷ UFC.mL⁻¹). Après 24 h d'incubation, les bactéries non adhérées ont été éliminées par lavage avec du sérum physiologique. Les épidermes ont été détachés des inserts avec un scalpel et introduits dans un récipient stérile contenant 9 mL de milieu MRS. Les bactéries adhérées ont été détachées par action mécanique (bains-ultrasons) et dénombrées par dilution-étalement sur milieu gélosé.

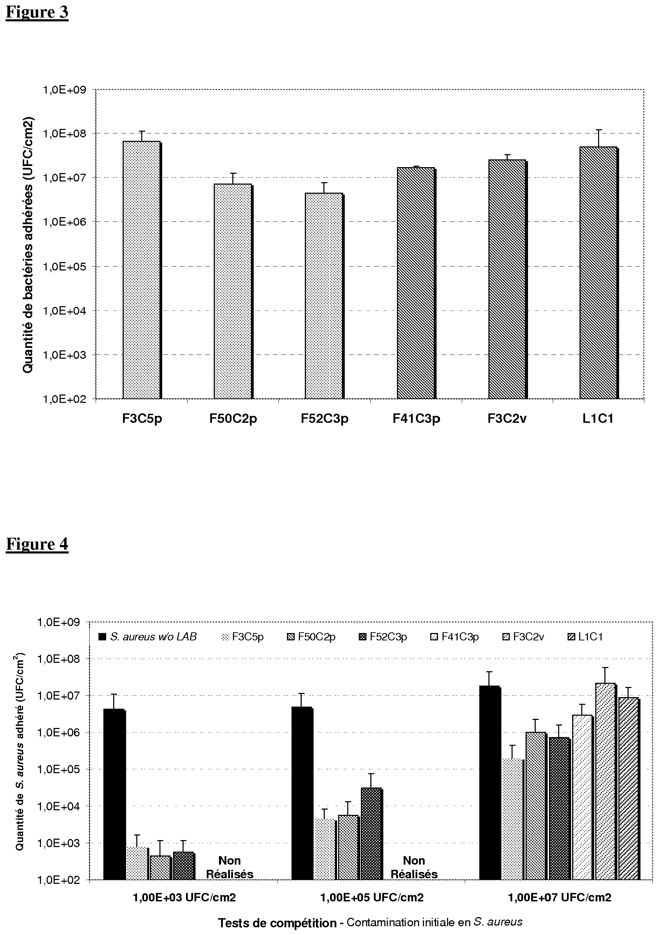

Les bactéries *Lactobacillus saniviri* F3C5p, *Lactobacillus salivarius* F50C2p, *Lactobacillus salivarius* F52C3p, *Lactobacillus salivarius* F41C3p, *Streptococcus mitis* F3C2v et *Lactobacillus pentosus* ou *plantarum* L1C1 sont capables d'adhérer sur l'épiderme et de persister pendant 24 heures. La souche *Lactobacillus saniviri* F3C5p présente la capacité d'adhésion la plus importante (Cf. figure 3).

### EXEMPLE 4 : Effet barrière - inhibition de l'adhésion des pathogènes sur une surface collagène

La capacité des souches de bactéries *Lactobacillus saniviri* F3C5p, *Lactobacillus salivarius* F50C2p, *Lactobacillus salivarius* F52C3p, *Lactobacillus salivarius* F41C3p, *Streptococcus mitis* F3C2v et *Lactobacillus pentosus* ou *plantarum* L1C1 à limiter et/ou inhiber l'adhésion d'espèces pathogènes (*S. aureus* MRSA ATCC 43300 ou *P. aeruginosa* ATCC 9027) sur des matrices contenant du collagène a été évaluée. Les tests ont été menés dans des microplaques 24 puits coatées avec du collagène de type I (BD Biocoat™ Collagen I).

Les bactéries pathogènes ont été cultivées pendant 16 heures à 37°C dans du milieu Tryptic Soy Broth (TSB). Après incubation, les cultures ont été diluées dans du milieu TSB à des concentrations d'environ 2,5 × 10⁷ UFC.mL⁻¹, 2,5 × 10⁵ UFC.mL⁻¹ et 2,5 × 10³ UFC.mL⁻¹. Les bactéries *Lactobacillus saniviri* F3C5p, *Lactobacillus salivarius* F50C2p, *Lactobacillus salivarius* F52C3p, *Lactobacillus salivarius* F41C3p, *Streptococcus mitis* F3C2v et *Lactobacillus pentosus* ou *plantarum* L1C1 ont été cultivées dans du milieu de Man Rogosa Sharpe (MRS) pendant 16 heures, à 37°C et en anaérobiose.

Pour les tests de compétition d'adhésion, les bactéries F3C5p, F50C2p et F52C3p et la bactérie pathogène (*S. aureus* ou *P. aeruginosa*) ont été ajoutées simultanément à une concentration respective d'environ 2,5 × 10⁷ UFC.mL⁻¹ et 2,5 × 10⁷ UFC.mL⁻¹ ou 2,5 × 10⁵ UFC.mL⁻¹ ou 2,5 × 10³ UFC.mL⁻¹ pour la bactérie pathogène. Les bactéries F41C3p, F3C2v et L1C1 et la bactérie pathogène (*S. aureus* ou *P. aeruginosa*) ont été ajoutées simultanément à une concentration respective d'environ 2,5 × 10⁷ UFC.mL⁻¹ et 2,5 × 10⁷ UFC.mL⁻¹.

Pour les tests d'exclusion, les bactéries F3C5p, F50C2p et F52C3p ont été introduites dans les puits à une concentration d'environ 2,5 × 10⁷ UFC.mL⁻¹ et incubées à 37°C pendant 24 heures. Après adhésion, les puits ont été lavés avec du sérum physiologique et la souche pathogène (*S. aureus* ou *P. aeruginosa*) a été introduite dans les puits à des concentrations d'environ 2,5 × 10⁷ UFC.mL⁻¹ ou 2,5 × 10⁵ UFC.mL-¹ ou 2,5 × 10³ UFC.mL⁻¹. Les bactéries F41C3p, F3C2v et L1C1 ont été testées à une concentration d'environ 2,5 × 10⁷ UFC.mL⁻¹ avec la souche pathogène (*S. aureus* ou *P. aeruginosa*) à une concentration d'environ 2,5 × 10⁷ UFC.mL⁻¹.

Pour chacun des tests, après 24 heures de contact, les bactéries pathogènes ont été spécifiquement dénombrées sur du milieu de Baird-Parker supplémenté avec une émulsion de jaune d'oeuf au tellurite de potassium pour *S. aureus* ou du milieu Céphalosparine / Fucidine / Cetrimide (CFC) pour *P. aeruginosa.* Après 24 h d'incubation à 35°C ± 2°C, les colonies caractéristiques ont été dénombrées. Un témoin non traité avec les bactéries selon l'invention a été réalisé pour chacun des tests.

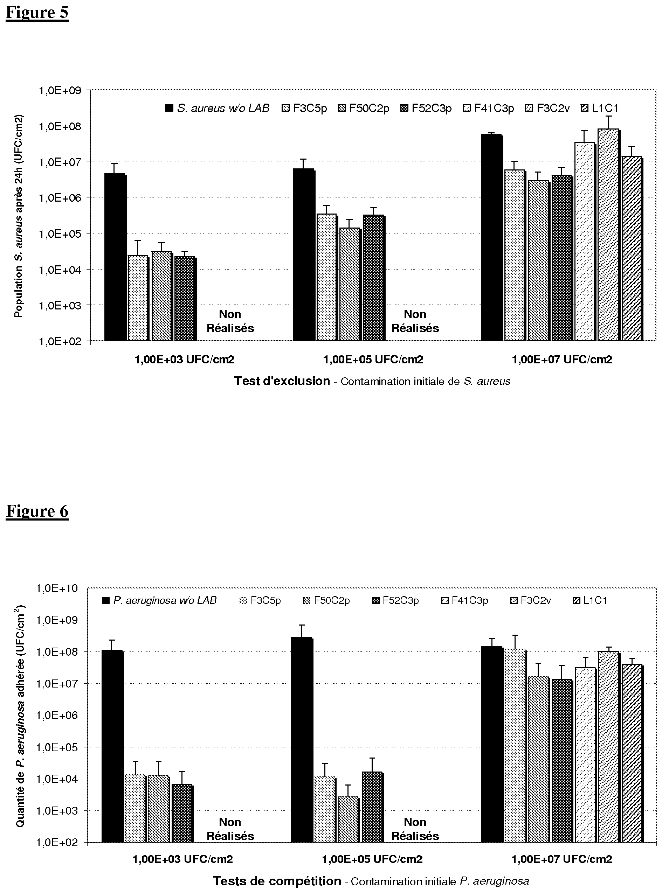

Les bactéries F3C5p, F50C2p, F52C3p, F41C3p, F3C2v et L1C1 permettent de limiter fortement l'adhésion de *S. aureus* et la colonisation du support coaté avec du collagène par rapport au témoin non-traité pour les trois concentrations testées (Cf. figures 4 et 5).

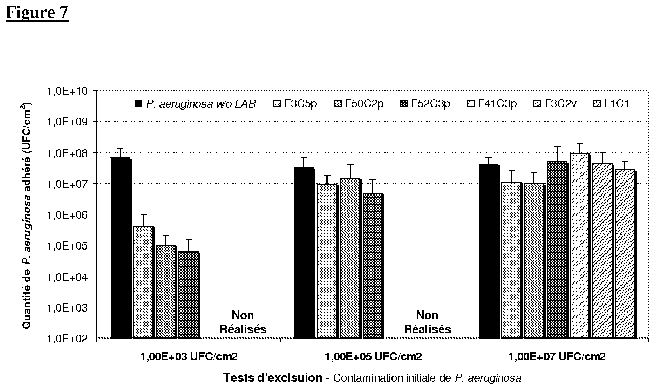

Les bactéries F3C5p, F50C2p, F52C3p, F41C3p, F3C2v et L1C1 permettent de limiter l'adhésion de *P. aeruginosa* et la colonisation du support coaté avec du collagène par rapport au témoin non-traité lorsque la contamination initiale est inférieure à 10⁵ UFC/cm² (Cf. figures 6 et 7).

### EXEMPLE 5 : activités immuno-modulatrices sur des macrophages

Les souches de *Lactobacillus saniviri* F3C5p, *Lactobacillus salivarius* F50C2p, *Lactobacillus salivarius* F52C3, *Lactobacillus pentosus*/*plantarum* L1C1, *Lactobacillus salivarius* F41C3p et *Streptococcus mitis* F3C2v ont été cultivées dans du milieu de Man Rogosa Sharpe (MRS) pendant 16 heures, à 37°C et en anaérobiose. Les bactéries ont été récupérées par centrifugation puis inactivées par des ultrasons ou par la chaleur (95°C) afin d'obtenir des extraits bactériens.

Des cellules monocytaires THP1 (ATCC TIB-202) ont été cultivées dans du milieu Roswell Park Memorial Institute medium (RPMI) et ont été différenciées en macrophages par addition de Phorbol-12-Myristate-13-Acétate (PMA). Après 24 heures de culture, les macrophages obtenus ont été exposés (i) soit aux extraits bactériens seuls, (ii) soit à du LPS d'*E.coli* O127:B8, (iii) soit à du LPS d'*E.coli* O127:B8 en présence d'extrait cellulaire. Après 3,5 heures de contact, la concentration en Facteur de nécrose tumorale (Tumor Necrosis Factor ou TNF-α) a été mesurée dans le surnageant de culture par une méthode ELISA.

L'ajout des extraits bactériens seuls n'induit pas de libération significative de TNF-α par rapport au témoin ne contenant pas d'extrait cellulaire et donc n'induit pas de réaction pro-inflammatoire, à l'exception de la souche L1C1 qui induit une faible libération de TNF- α (concentration de 121 pg/mL).

La stimulation des macrophages avec le LPS d'*E. coli* entraîne une libération importante de TNF-α en absence d'extraits bactériens (concentration de l'ordre de 500 pg/mL). L'ajout simultané des extraits cellulaires préparés à partir des différentes souches bactériennes (F3C5p, F41C3p, F50C2p, F52C3p, L1C1 ou F3C2v) avec le LPS permet de réduire significativement (facteur de 2 à 5 fois) la libération de TNF-α par les macrophages. Ces résultats démontrent le potentiel anti-inflammatoire des 6 souches de bactéries lactiques.

Les références citées dans la présente demande sont les suivantes:
1 Brachkova MI, Marques P, Rocha J, Sepodes B, Duarte MA, Pinto JF (2011). Alginate films containing Lactobacillus plantarum as wound dressing for prevention of burn infection. J. Hosp. Infect. 79: 375-377
2 Costerton, J.W., Stewart, P. S. and Greenberg, E. P. (1999). Bacterial biofilms: a common cause of persistent infections. Science 284 (5418): 1318-1322
3 Gan BS, Kim J, Reid G, Cadieux P, Howard JC (2002). Lactobacillus fermentum RC-14 inhibits Staphylococcus aureus infection of surgical implants in rats. J. Infect. Dis. 185: 1369-1372
4 Gueniche A, Hennino A, Goujon C, Dahel K, Bastien P, Martin R, Jourdain R, Breton L (2006). Improvement of atopic dermatitis skin symptoms by Vitreoscilla filiformis bacterial extract. Eur. J. Dermatol. 16: 380-384
5 Gueniche A, Buetler T, Benyacoub J, Blum S (2008a). Lactobacillus johnsonii provides a dose-dependent protection against UVR-induced immunosuppression. Eur. J. Dermatol. 18: 476-477
6 Gueniche A, Cathelineau AC, Bastien P, Esdaile J, Martin R, Queille Roussel C, Breton L (2008b). Vitreoscilla filiformis biomass improves seborrheic dermatitis. J. Eur. Acad. Dermatol. Venereol. 22: 1014-1015
7 Gueniche A, Dahel K, Bastien P, Martin R, Nicolas JF, Breton L (2008c). Vitreoscilla filiformis bacterial extract to improve the efficacy of emollient used in atopic dermatitis symptoms. J. Eur. Acad. Dermatol. Venereol. 22: 746-747
8 Gueniche A, Knaudt B, Schuck E, Volz T, Bastien P, Martin R, Rocken M, Breton L, Biedermann T (2008d). Effects of nonpathogenic gram-negative bacterium Vitreoscilla filiformis lysate on atopic dermatitis: a prospective, randomized, double-blind, placebo-controlled clinical study. Br. J. Dermatol. 159: 1357-1363
9 Gueniche A, Bastien P, Ovigne JM, Kermici M, Courchay G, Chevalier V, Breton L, Castiel-Higounenc I (2009). Bifidobacterium longum lysate, a new ingredient for reactive skin. Exp. Dermatol. 19: e1-8
10 Gueniche A, Benyacoub J, Philippe D, Bastien P, Kusy N, Breton L, Blum S, Castiel-Higounenc I (2010). Lactobacillus paracasei CNCM I-2116 (ST11) inhibits substance P-induced skin inflammation and accelerates skin barrier function recovery in vitro. Eur. J. Dermatol. 20: 731-737
11 Halper J, Leshin LS, Lewis SJ, Li WI (2003). Wound healing and angiogenic properties of supernatants from Lactobacillus cultures. Exp Biol Med 228: 1329-1337
12 Oh S, Kim SH, Ko Y, Sim JH, Kim KS, Lee SH, Park S, Kim YJ (2006). Effect of bacteriocin produced by Lactococcus sp. HY 449 on skin-inflammatory bacteria. Food and Chemical Toxicology 44 (2006) 1184-1190
13 Im E, Choi YJ, Kim CH, Fiocchi C, Pothoulakis C, Rhee SH (2009). The angiogenic effect of probiotic Bacillus polyfermenticus on human intestinal microvascular endothelial cells is mediated by IL-8. Am. J. Physiol. Gastrointest. Liver Physiol. 297: G999-G1008
14 Mi-Sun Kang MS, Oh JS, Lee SW, Lim HS, Choi NK, and Kim SM (2012). Effect of Lactobacillus reuteri on the proliferation of Propionibacterium acnes and Staphylococcus epidermidis. The Journal of Microbiology (2012) Vol. 50, No. 1, pp. 137-142
15 Peral MC, Martinez MA, Valdez JC (2009). Bacteriotherapy with Lactobacillus plantarum in burns. Int. Wound J. 6: 73-81
16 Peral MC, Rachid MM, Gobbato NM, Huaman Martinez MA, Valdez JC (2010). Interleukin-8 production by polymorphonuclear leukocytes from patients with chronic infected leg ulcers treated with Lactobacillus plantarum. Clin. Microbiol. Infect. 16: 281-286
17 Ramos AN, Gobbato N, Rachid M, Gonzalez L, Yantorno O, Valdez JC (2010). Effect of Lactobacillus plantarum and Pseudomonas aeruginosa culture supernatants on polymorphonuclear damage and inflammatory response. Int. Immunopharmacol. 10: 247-251
18 Sikorska H, Smoragiewiczb W (2013). Role of probiotics in the prevention and treatment of meticillin-resistant Staphylococcus aureus infections. International Journal of Antimicrobial Agents 42 (2013) 475-481
19 Valdez JC, Peral MC, Rachid M, Santana M, Perdigon G (2005). Interference of Lactobacillus plantarum with Pseudomonas aeruginosa in vitro and in infected burns: the potential use of probiotics in wound treatment. Clin. Microbiol. Infect. 11: 472-479
20 Alberto N. Ramos, María E. Sesto Cabral, Diego Noseda, Alejandra Bosch, Osvaldo M. Yantorno, Juan C. Valdez, (2012). Antipathogenic properties of Lactobacillus plantarum on Pseudomonas aeruginosa: The potential use of its supernatants in the treatment of infected chronic wounds. Wound Rep Reg (2012)

## Revendications

1. Bactérie choisie parmi *Lactobacillus saniviri* enregistrée le 12 juillet 2012 à la CNCM sous le n° 1-4650, *Lactobacillus salivarius* enregistrées le 12 juillet 2012 à la CNCM respectivement sous les n° 1-4651, 1-4652 et 1-4653, *Streptococcus mitis* enregistrée le 12 juillet 2012 sous le n° CNCM 1-4654 et *Lactobacillus pentosus* ou *plantarum* enregistrée le 12 juillet 2012 sous le n° CNCM 1-4655.

2. Bactérie selon la revendication 1 pour son utilisation comme principe actif, dispositif médical, cosmétique ou complément alimentaire ou comme principe actif au sein d'un médicament, d'un dispositif médical, d'un cosmétique ou d'un complément alimentaire.

3. Bactérie selon l'une des revendications 1 ou 2 pour son utilisation pour prévenir et/ou traiter une infection et/ou une colonisation liée à au moins une bactérie ou une levure choisie parmi *S*. *aureus, P. aeruginosa, Streptococcus pyogenes, Enterococcus faecium, Enterobacter cloacae, Proteus mirabilis, Bacteroides fragilis, Staphylococcus epidermidis, Propionibacterium acnes, Candida albicans* et *Malassezia furfur.*

4. Bactérie selon l'une des revendications 1 à 3 pour son utilisation pour prévenir et/ou traiter une infection et/ou une colonisation liée à *S*. *aureus* et/ou *P. aeruginosa.*

5. Bactérie selon l'une des revendications 1 à 3 pour son utilisation comme immuno-modulateur.

6. Bactérie selon l'une des revendications 1 à 4 **caractérisée en ce qu'**elle est appliquée sur la peau, les plaies, les muqueuses et/ou les phanères.

7. Bactérie selon la revendication 3 ou 4, **caractérisée en ce que** l'infection et/ou la colonisation concerne la peau, une plaie, en particulier une plaie choisie parmi les ulcères du pied diabétique, les ulcères de jambe d'origine artérielle, les ulcères de jambe d'origine veineuse, les escarres, les panaris, les plaies aiguës, les plaies traumatiques, et les plaies post-opératoires.

8. Composition comprenant au moins une bactérie selon la revendication 1.

9. Composition selon la revendication 8, **caractérisée en ce qu'**elle comprend en outre au moins un composé choisi parmi les probiotiques, les prébiotiques et les levures.

10. Composition selon l'une des revendications 8 ou 9, **caractérisée en ce qu'**elle comprend en outre au moins un actif choisi parmi les antifongiques, les antidouleurs, les anti-inflammatoires, les actifs favorisant la cicatrisation, les agents hydratants, les agents kératolytiques, les actifs restructurants et les anesthésiques.

11. Composition selon l'une des revendications 8 à 10, **caractérisée en ce qu'**elle est adaptée pour une application topique, orale ou parentérale.

12. Composition selon l'une des revendications 8 à 11, **caractérisé en ce qu'**elle se présente sous forme d'onguent, de crème, de lait, de pommade, de poudre, de tampon imbibé, de syndet, de lingette, de solution, de gel, de spray, de mousse, de suspension, de lotion, de stick, de shampoing, de base lavante, comprimé, gélule, d'un produit alimentaire, ou solution injectable.

13. Composition selon l'une des revendications 8 à 12, **caractérisée en ce que** la bactérie est présente en quantité comprise entre 10³ à 10¹² bactéries, de préférence 10⁶ à 10¹¹.

14. Composition selon l'une des revendications 8 à 13, **caractérisée en ce que** la bactérie est présente sous forme de cellule inactivée, de cellule vivante, de lysat cellulaire, encapsulée ou non, immobilisée ou non, lyophilisée ou non.

15. Pansement comprenant au moins une bactérie selon la revendication 1, ou au moins une composition selon l'une des revendications 8 à 14.

## Patentansprüche

1. Bakterium, ausgewählt aus *Lactobacillus saniviri,* hinterlegt am 12. Juli 2012 bei der CNCM unter der Nr. I-4650, *Lactobacillus salivarius,* hinterlegt am 12. Juli 2012 bei der CNCM unter der Nr. I-4651, I-4652 bzw. I-4653, *Streptococcus mitis,* hinterlegt am 12. Juli 2012 unter der Nr. CNCM I-4654 und *Lactobacillus peutosus* oder *plautarum,* hinterlegt am 12. Juli 2012 unter der Nr. CNCM I-4655.

2. Bakterium nach Anspruch 1, zur Verwendung als aktiver Wirkstoff, Medizinprodukt, Kosmetik oder Nahrungsergänzung oder als aktiver Wirkstoff in einem Medikament, einem Medizinprodukt, einer Kosmetik oder einer Nahrungsergänzung.

3. Bakterium nach einem der Ansprüche 1 oder 2, zur Verwendung zur Vorbeugung und/oder Behandlung einer Infektion und/oder einer Besiedelung verbunden mit wenigstens einem Bakterium oder einer Hefe, ausgewählt aus *S*. *aureus, P. aeruginosa, Streptococcus pyogenes, Enterococcus faecium, Enterobacter cloacae, Proteus mirabilis, Bacteroides fragilis, Staphylococcus epidermidis, Propionibacterium acnes, Caudida albicans* und *Malassezia furfur.*

4. Bakterium nach einem der Ansprüche 1 bis 3, zur Verwendung zur Vorbeugung und/oder Behandlung einer Infektion und/oder Besiedelung verbunden mit *S*. *aureus* und/oder *P. aeruginosa.*

5. Bakterium nach einem der Ansprüche 1 bis 3, zur Verwendung als Immunmodulator.

6. Bakterium nach einem der Ansprüche 1 bis 4, **dadurch gekennzeichnet, dass** es auf der Haut, den Wunden, den Schleimhäuten und/oder den Hautanhangsgebilden angewendet wird.

7. Bakterium nach Anspruch 3 oder 4, **dadurch gekennzeichnet, dass** die Infektion und/oder die Besiedelung die Haut, eine Wunde, insbesondere eine Wunde ausgewählt aus diabetischen Fußulcera, Beinulcera arteriellen Ursprungs, Beinulcera venösen Ursprungs, Schorf, Paronychie, akuten Wunden, traumatischen Wunden und post-operativen Wunden betrifft.

8. Zusammensetzung, umfassend wenigstens ein Bakterium nach Anspruch 1.

9. Zusammensetzung nach Anspruch 8, **dadurch gekennzeichnet, dass** sie weiterhin wenigstens eine Zusammensetzung umfasst, die ausgewählt ist aus Probiotika, Prebiotika und Hefen.

10. Zusammensetzung nach Anspruch 8 oder 9, **dadurch gekennzeichnet, dass** sie weiterhin wenigstens einen Wirkstoff umfasst, der ausgewählt ist aus Fungiziden, Schmerzmitteln, entzündungshemmenden Mitteln, Mitteln zur Förderung der Wundheilung, Hydratisierungsmitteln, keratolytischen Mitteln, restrukturierenden Wirkstoffen und Betäubungsmitteln.

11. Zusammensetzung nach einem der Ansprüche 8 bis 10, **dadurch gekennzeichnet, dass** sie für die topische, orale oder parenterale Anwendung geeignet ist.

12. Zusammensetzung nach einem der Ansprüche 8 bis 11, **dadurch gekennzeichnet, dass** sie als Salbe, Creme, Milch, Wundsalbe, Pulver, getränkter Bausch, Syndet, Feuchttuch, Lösung, Gel, Spray, Schaum, Suspension, Lotion, Stift, Shampoo, Waschgrundlage, Tablette, Kapsel, Nahrungsprodukt oder Injektionslösung vorliegt.

13. Zusammensetzung nach einem der Ansprüche 8 bis 12, **dadurch gekennzeichnet, dass** das Bakterium in einer Menge zwischen 10³ und 10¹² Bakterien vorliegt, vorzugsweise 10⁶ bis 10¹¹.

14. Zusammensetzung nach einem der Ansprüche 8 bis 13, **dadurch gekennzeichnet, dass** das Bakterium in Form inaktivierter Zellen, lebender Zellen, als Zelllysat, eingekapselt oder nicht eingekapselt, immobilisiert oder nicht immobilsiert, lyophilisiert oder nicht lyophilisiert vorliegt.

15. Pflaster, umfassend wenigstens ein Bakterium nach Anspruch 1 oder wenigstens eine Zusammensetzung nach einem der Ansprüche 8 bis 14.

## Claims

1. Bacteria chosen from among *Lactobacillus saniviri* registered on 12 July 2012 at CNCM as No. 1-4650, *Lactobacillus salivarius* registered on 12 July 2012 at CNCM as Numbers 1-4651, 1-4652 et 1-4653 respectively, *Streptococcus mitis* registered on 12 July 2012 as CNCM No. 1-4654 and *Lactobacillus pentosus* or *plantarum* registered on 12 July 2012 as CNCM No. 1-4655.

2. Bacteria according to claim 1 for use as active constituent, medical device, cosmetic or as a food supplement or active constituent in a medicine, a medical device, a cosmetic or food supplement.

3. Bacteria according to either claim 1 or 2 for use to prevent and/or treat an infection and/or colonisation related to at least one bacterium or a yeast chosen from among *S*. *aureus, P. aeruginosa, Streptococcus pyogenes. Euterococcus faecium, Euterobacter cloacae, Proteus mirabilis, Bacteroides fragilis, Staphylococcus epidermidis, Propionibacterium acnes, Candid albicans* and *Malassezia furfur.*

4. Bacteria according to one of claims 1 to 3 for use to prevent and/or treat an infection and/or a colonisation related to *S*. *aureus* and/or *P. aeruginosa.*

5. Bacteria according to one of claims 1 to 3 for use as an immunomodulator.

6. Bacteria according to one of claims 1 to 4 **characterised in that** it is applied on the skin, on wounds, mucous membranes and/or keratinous appendages.

7. Bacteria according to either claim 3 or 4, **characterised in that** the infection and/or colonisation relates to the skin, a wound, particularly a wound chosen from among diabetic foot ulcers, leg ulcers due to arterial disorders, leg ulcers due to venous disorders, bed sores, les panaritia, acute wounds, trauma wounds, and post-operative wounds.

8. Composition comprising at least one bacterium according to claim 1.

9. Composition according to claim 8, **characterised in that** it also comprises a least one compound chosen from among probiotics, prebiotics and yeasts.

10. Composition according to either claim 8 or 9, **characterised in that** it also comprises an active constituent chosen from among antifungals, pain killers, anti-inflammatories, active constituents conducive to healing, moisturising agents, keratolytic agents, restructuring agents and anaesthetics.

11. Composition according to one of claims 8 to 10, **characterised in that** it is adapted for topical, oral or parenteral application.

12. Composition according to one of claims 8 to 11, **characterised in that** it is presented in the form of a salve, cream, milk, ointment, powder, wet swab, syndet, wipe, solution, gel, spray, foam, suspension, lotion, stick, shampoo, cleansing base, tablet, capsule, food product or injectable solution.

13. Composition according to one of claims 8 to 12, **characterised in that** bacterium is present in a quantity of between 10³ to 10¹² bacteria, and preferably between 10⁶ to 10¹¹.

14. Composition according to one of claims 8 to 13, **characterised in that** the bacterium is present in the form of inactive cell, living cell, cell lysate, encapsulated or not, immobilised or not, freeze-dried or not.

15. Dressing comprising at least one bacterium according to claim 1, or at least one composition according to one of claims 8 to 14.
